# EUROPEAN PATENT SPECIFICATION

(11) **EP 2 206 519 B1**
(45) Date of publication and mention of the grant of the patent: **01.08.2012**
(21) Application number: 08752515.0
(22) Date of filing: 09.05.2008
(51) Int. Cl.: A61K 47/32, A61K 9/70, A61K 47/10, A61K 47/26, A61K 47/34

(54) **MEDICINAL PREPARATION FOR ORAL ADMINISTRATION**
MEDIZINISCHE ZUBEREITUNG ZUR ORALEN VERABREICHUNG
PRÉPARATION MÉDICINALE POUR ADMINISTRATION ORALE

(30) Priority: 28.09.2007 JP 2007255977
(43) Date of publication of application: 14.07.2010
(73) Proprietor: Lintec Corporation, Tokyo 173-0001 (JP)
(72) Inventor: SUGIURA, Yusaku, Tokyo 173-0001 (JP); KABUTO, Akio, Tokyo 173-0001 (JP); SUZUKI, Eiji, Tokyo 173-0001 (JP)
(74) Representative: Solf, Alexander
(86) International application number: PCT/JP2008/058632
(87) International publication number: WO 2009/041111

(56) References cited:
- EP-A1- 0 781 546
- EP-A1- 1 391 212
- EP-A1- 1 736 143
- EP-A1- 1 757 273
- EP-A1- 1 757 308
- EP-A1- 2 133 060
- EP-A1- 2 138 168
- JP-A- 2005 298 471
- JP-A- 2006 137 717
- JP-A- 2006 160 617
- JP-A- 2007 254 340
- JP-A- 2007 254 341
- JP-T- 2007 520 517
- US-A- 6 153 222

## Description

### Technical Field

The present invention relates to an orally-administered agent.

### Related Art

As examples of an orally-administered agent containing a medicine, there are known solid formulations and jelly-like (or gel-like) semisolid formulations. The solid formulations (e.g., tablets and capsules) are usually hard to take as they stand and therefore need to be taken together with a large quantity of water. It is often difficult for aged persons or infants to take the solid formulations. In addition, the solid formulations have a risk that they are likely to get stuck in a trachea or may adhere to an esophagus.

In contrast, the jelly-like semisolid formulations are easy to swallow and can be easily taken by the aged persons or the infants. However, the semisolid formulations contain a large quantity of moisture and have a drawback in that the medicine contained therein is susceptible to decomposition or degradation. Moreover, there is a need to prevent infiltration of bacteria when producing and storing the semisolid formulations. This makes it cumbersome and complicated to handle the semisolid formulations. For these reasons stated above, the semisolid formulations involve a difficulty in realizing widespread use.

In view of the problems noted above, a study has been made in recent years on film formulations having water solubility (see, e.g., the following Patent Document). Within an oral cavity, the film formulations are dissolved by saliva or gelatinized by absorbing water. Therefore, it is relatively easy to swallow the film formulations. In addition, there is no need to add moisture into the film formulations. This makes it easy to handle the film formulations when producing and storing the same.

However, it is sometimes a case that aged persons or patients suffering from salivary secretion disorders undergo reduced saliva secretion. In this case, the film formulations are unable to sufficiently absorb the saliva, which makes it difficult to easily and rapidly swallow the film formulations. Another problem resides in that it is impossible to apply or use a medicine which tends to give unpleasant tastes (e.g., a bitter taste, an astringent taste and a hot taste) or offensive odors when the film formulations are dissolved within the oral cavity.

The Patent Document is JP-A 11-116469 as an example of related art.

### SUMMARY OF THE INVENTION

It is an object of the present invention to provide an orally-administered agent that can be rapidly gelatinized with a small quantity of water and can be swallowed with ease.

According to the present invention, it is possible to provide an orally-administered agent comprised of a laminated body having surface layers. The laminated body comprises: a medicine-containing layer containing a medicine and having surfaces; and at least one gel-forming layer provided on one of the surfaces of the medicine-containing layer as one of the surface layers of the laminated body, wherein the at least one gel-forming layer includes a gel-forming agent to be swelled and gelatinized when absorbing a water-based liquid. A water absorption speed per 1 g of the gel-forming layer with respect to water of 37°C is 0.5 g/(g·s) or higher, and a gel fraction of the gel-forming layer is 20 mass% or more when the gel-forming layer is dipped into an aqueous solution of 0.9 mass% of sodium chloride having a temperature of 37°C.

According to such a present invention, it is possible to provide an orally-administered agent that can be rapidly gelatinized with a small quantity of water and can be swallowed with ease.

In the orally-administered agent according to the present invention, it is preferred that the gel-forming agent includes a carboxyl group-containing polymer.

In the orally-administered agent according to the present invention, it is also preferred that the carboxyl group-containing polymer has a property that a viscosity of an aqueous solution of 0.2 mass% is in the range of 1500 to 50000 mPa·s at 20°C.

In the orally-administered agent according to the present invention, it is also preferred that the gel-forming layer includes a water absorption promoter for promoting absorption of the water-based liquid into the gel-forming layer.

In the orally-administered agent according to the present invention, it is also preferred that a content of the water absorption promoter included in the gel-forming layer is in the range of 1 to 20 mass%.

In the orally-administered agent according to the present invention, it is also preferred that the water absorption promoter has a property that a viscosity of an aqueous solution of 5 mass% is in the range of 0.3 to 5.0 mPa·s at 37°C.

In the orally-administered agent according to the present invention, it is also preferred that the water absorption promoter includes glycerin, and a content of the glycerin included in the water absorption promoter is in the range of 35 to 95 mass%.

In the orally-administered agent according to the present invention, it is also preferred that the water absorption promoter includes a solid-state compound in an atmosphere of 1 atm at 25°C.

In the orally-administered agent according to the present invention, it is also preferred that the water absorption promoter includes at least one of a sugar and a glycol.

In the orally-administered agent according to the present invention, it is also preferred that the glycol includes a compound having an ethylene glycol chain in a chemical structure thereof.

In the orally-administered agent according to the present invention, it is also preferred that the glycol includes a surfactant of which hydrophile-lipophile balance (HLB) is in the range of 15 to 20.

In the orally-administered agent according to the present invention, it is also preferred that a mass-average molecular weight of the glycol is in the range of 600 to 35000.

In the orally-administered agent according to the present invention, it is also preferred that the gel-forming layer has an outer surface, and the outer surface has convex portions.

In the orally-administered agent according to the present invention, it is also preferred that the laminated body is formed into a film shape.

### BRIEF DESCRIPTION OF THE DRAWINGS

FIG. 1 is a section view showing an orally-administered agent in accordance with a first embodiment of the present invention.
FIG. 2 is a section view showing an orally-administered agent in accordance with a second embodiment of the present invention.
FIG. 3 is a section view showing an orally-administered agent in accordance with one example of other embodiments of the present invention.
FIG. 4 is a section view showing an orally-administered agent in accordance with another example of other embodiments of the present invention.

### DETAILED DESCRIPTION OF THE PREFERRED EMBODIMENTS

Hereinafter, the present invention will be described in detail based on certain preferred embodiments.

An orally-administered agent of the present invention may have any shape. The following description will be made on the assumption that the present orally-administered agent is a film-like formulation (or a sheet-like formulation).

Now, different embodiments of the present invention will be described with reference to the accompanying drawings.

### <First Embodiment>

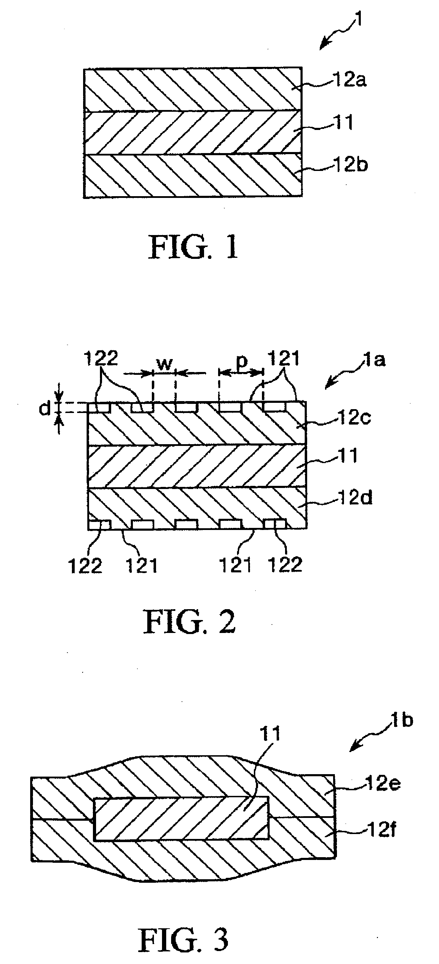

FIG. 1 is a section view showing an orally-administered agent in accordance with a first embodiment of the present invention.

As shown in FIG. 1, the orally-administered agent 1 of the first embodiment is formed of a laminated body including a medicine-containing layer 11 which contains a medicine, a gel-forming layer 12a laminated on one major surface of the medicine-containing layer 11 and a gel-forming layer 12b laminated on the other major surface of the medicine-containing layer 11. The gel-forming layers 12a and 12b are directly laminated on the respective major surfaces of the medicine-containing layer 11, in which state they serve as outer surface layers of the orally-administered agent 1. Each of the gel-forming layers 12a and 12b has a flat major surface that forms the corresponding outer surface of the orally-administered agent 1.

The orally-administered agent 1 is a film-like formulation (or a sheet-like formulation). If the film-like formulation is used as the orally-administered agent 1, it is possible to reduce a moisture content in the film formulation. This makes it possible to enhance stability of the medicine (especially, an easily-hydrolysable medicine) contained in the medicine-containing layer 11, as compared to a jelly-like formulation containing a large quantity of moisture. Moreover, the film formulation is easy to handle, and assists in reducing a formulation packing cost.

The gel-forming layers 12a and 12b provided on the surfaces of the orally-administered agent 1 are swelled and gelatinized by moisture such as saliva within an oral cavity of a patient. Thus, the orally-administered agent 1 is changed into a dosage form having an easy-to-swallow size, shape, elasticity and viscosity. This enables the patient to take the orally-administered agent 1 with ease. In addition, it is possible to reduce a risk that the orally-administered agent 1 is likely to get stuck in a trachea when taken by the patient. This makes it possible for the patient, whether an aged person or an infant, to safely take the orally-administered agent 1.

Furthermore, the gel-forming layers 12a and 12b provided on the surfaces of the orally-administered agent 1 can prevent the medicine contained in the medicine-containing layer 11 from being dissolved within the oral cavity, thereby masking the medicine tastes (e.g., a bitter taste, an astringent taste and a hot taste) or the medicine odors.

Next, the respective layers of the orally-administered agent 1 will be described in more detail.

### <Medicine-containing Layer>

The medicine-containing layer 11 is a layer containing the medicine to be administered.

The medicine contained in the medicine-containing layer 11 is a medicine to be administered to a patient. Such a medicine is not limited to a specific one but may be any orally-administrable medicine. Examples of the orally-administrable medicine include: medicines acting on a central nerve, including a hypnotic medicine such as amobarbital, estazoram, triazolam, nitrazepam, pentobarbital or the like, a psychotropic medicine such as amitriptyline hydrochloride, imipramine hydrochloride, oxazolam, chlordiazepoxide, chlorpromazine, diazepam, sulpiride, haloperidol or the like, an antiparkinson medicine such as trihexyphenidyl, levodopa or the like, an analgesic medicine and an anti-inflammatory medicine such as aspirin, isopropylantipyrine, indometacin, diclofenac sodium, mefenamic acid, streptokinase, streptodornase, serrapeptase, pronase or the like and a central nervous metabolic activation medicine such as APT, vinpocetine or the like; medicines acting on a respiratory organ, including an expectorant medicine such as carbocysteine, bromhexine hydrochloride or the like and an antiasthmatic medicine such as azelastine hydrochloride, oxatomide, theophylline, terbutaline sulfate, tranilast, procaterol hydrochloride, ketotifen fumarate or the like; medicines acting on a circulatory system, including a cardiac stimulant such as aminophylline, digitoxin, digoxin or the like, an antiarrhythmic medicine such as ajmaline, disopyramide, procainamide hydrochloride, mexiletine hydrochloride or the like, an antianginal medicine such as amyl nitrite, alprenolol hydrochloride, isosorbide dinitrate, nicorandil, oxyfedrine, dipyridamole, dilazep hydrochloride, diltiazem hydrochloride, nitroglycerin, nifedipine, verapamil hydrochloride or the like, a peripheral vasodilator such as kallidinogenase or the like, an antihypertensive medicine such as atenolol, captopril, clonidine hydrochloride, metoprolol tartrate, spironolactone, triamterene, trichlormethiazide, nicardipine, hydralazine hydrochloride, hydrochlorothiazide, prazosin hydrochloride, furosemide, propranolol hydrochloride, enalapril maleate, methyldopa, labetalol hydrochloride, reserpine or the like and an antiarteriosclerotic medicine such as clofibrat, dextran sulfate, nicomol, niceritrol or the like; blood and hematological medicines, including a hemostatic medicine such as carbazochrome sodium sulfonate, tranexamic acid or the like, an antithrombogenic medicine such as ticlopidine hydrochloride, warfarin potassium or the like and an anemia medicine such as ferric sulfate or the like; medicines acting on a gastrointestinal system, including an antiulcer medicine such as azulene, aldioxa, cimetidine, ranitidine hydrochloride, famotidine, teprenone, rebamipide or the like, an antiemetic medicine such as domperidone, metoclopramide or the like, a cathartic medicine such as sennoside, digestive enzyme preparations, and a therapeutic medicine for liver diseases such as glycyrrhizin, liver extract preparations or the like; medicines acting on a metabolic disease, including an antidiabetic medicine such as glibenclamide, chlorpropamide, tolbutamide or the like and an antipodagric medicine such as allopurinol, colchicines or the like; medicines for an ophthalmic field, including acetazolamide; medicines for an otological field, including an anti-vertigo medicine such as difenidol hydrochloride, betahistine mesylate or the like; chemotherapeutic medicines and antibiotic medicines including isoniazid, ethambutol hydrochloride, ofloxacin, erythromycin stearate, cefaclor, norfloxacin, fosfomycin calcium, minocycline hydrochloride, rifampicin, rokitamycin or the like; antineoplastic medicines including cyclophosphamide, tegafur or the like; immunosuppressive medicines including azathioprine; hormones and endocrine medicines including progestational hormone, salivary hormone, thiamazole, prednisolone, betamethasone, liothyronine, levothyroxine or the like; and physiologically active substances (autacoids) including an antihistamine medicine such as diphenhydramine hydrochloride, clemastine fumarate, D-chlorpheniramine maleate or the like and a vitamin such as alfacalcidol, cobamamide, tocopherol nicotinate, mecobalamin or the like. One or more of these medicines may be used independently or in combination according to the purposes of treatment and prevention.

Various kinds of medicines including a medicine administered in a small quantity and a medicine administered in a large quantity can be contained in the medicine-containing layer 11. In this regard, the medicine administered in a small quantity means a medicine whose one-time dosage amount is 1 mg or less, while the medicine administered in a large quantity means a medicine whose one-time dosage amount is 300 mg or more.

A content of the medicine in the medicine-containing layer 11 is not particularly limited and may be suitably adjusted depending on a kind of the medicine and the volume of the medicine-containing layer 11. The medicine content is preferably in the range of 0.01 to 70 mass%, more preferably in the range of 0.01 to 40 mass% and even more preferably in the range of 0.01 to 35 mass%. This makes it possible to have a sufficiently quantity of the medicine contained in the orally-administered agent 1 while enhancing physical strength of the orally-administered agent 1.

The orally-administered agent 1 exhibits great enough physical strength even when a relatively large quantity of the medicine is contained in the medicine-containing layer 11 or when an insoluble and bulky medicine having a tendency to reduce the physical strength of the medicine-containing layer 11 is contained in the medicine-containing layer 11. Presumably, this is because the orally-administered agent 1 includes the gel-forming layers 12a and 12b provided on the both surfaces of the medicine-containing layer 11 and because the gel-forming layers 12a and 12b impart great enough physical strength to the orally-administered agent 1.

The medicine-containing layer 11 may include a base (namely, a base agent for the medicine-containing layer) which serves to keep the administered medicine in a desired state in the medicine-containing layer 11 and to adjust the shape and the physical strength of the medicine-containing layer 11. Examples of the base used in the medicine-containing layer 11 include, but are not limited to: cellulose such as crystalline cellulose, carboxymethyl cellulose, hydroxypropyl cellulose, hydroxypropyl methyl cellulose, methyl cellulose, ethyl cellulose, acetyl cellulose, cellulose acetate phthalate, hydroxypropylmethyl cellulose phthalate, hydroxypropylmethyl cellulose acetate sucinate and carboxymethylethyl cellulose; derivatives of cellulose or pharmaceutically acceptable salts of cellulose (e.g., sodium salt); starch such as a-starch, oxidized starch, carboxymethyl starch sodium, hydroxypropyl starch, dextrin and dextran; derivatives of starch; sugars such as saccharose, maltose, lactose, glucose, fructose, pullulan, xanthane gum, cyclodextrin, xylitol, mannitol and sorbitol; acrylate derivatives such as a dimethylaminoethyl methacrylate-methacrylic acid copolymer, a methacrylic acid-ethylacrylate copolymer, a methacrylic acid-methylmethacrylate copolymer, an ethylmethacrylate-chlorotrimethylammonium methacrylic acid copolymer, a dimethylaminoethyl methacrylate-chloromethyl methacrylate copolymer and a methacrylic acid-chloroethyl acrylate copolymer; Sellac; polyvinylacetal diethylamino acetate; polyvinyl acetate; polyvinyl alcohol; polyvinyl pyrolidone; a vinylacetate-vinylpyrrolidone copolymer; natural rubbers such as Arabic gum and tragacanth gum; polyglucosamines such as chitin and chitosan; proteins such as gelatin, casein and soybean protein; titanium oxide; calcium monohydrogen phosphate; calcium carbonate; talc; stearic acid; magnesium aluminometasilicate; magnesium silicate; and silicic anhydride. One or more of these bases may be used independently or in combination according to the purposes.

A content of the base in the medicine-containing layer 11 is not particularly limited but may be preferably in the range of 30 to 99.9 mass%, more preferably in the range of 60 to 99.9 mass% and even more preferably in the range of 65 to 99.0 mass%. This makes it possible to easily and sufficiently enhance the physical strength of the medicine-containing layer 11 while allowing a sufficiently quantity of the medicine to be contained in the medicine-containing layer 11.

A thickness of the medicine-containing layer 11 can be suitably adjusted within a range permitting an oral administration. The thickness of the medicine-containing layer 11 is not particularly limited but may be preferably in the range of 0.5 to 1000 µm and more preferably in the range of 10 to 500 µm. This makes it possible to sharply reduce variations in the medicine content and thickness which would occur in respective portions of the medicine-containing layer 11. In addition, this makes it possible to sufficiently increase overall softness of the orally-administered agent 1 and to greatly enhance ease of taking the orally-administered agent 1.

### <Gel-forming Layer>

The gel-forming layers 12a and 12b are layers that can be swelled and gelatinized by absorbing a water-based liquid. The gel-forming layer 12a is provided on one major surface of the medicine-containing layer 11 to form one outermost layer of the orally-administered agent 1. The gel-forming layer 12b is provided on the other major surface of the medicine-containing layer 11 to form the other outermost layer of the orally-administered agent 1.

Only the gel-forming layer 12a will be representatively described below, because the gel-forming layers 12a and 12b have substantially the same configuration.

In the present invention, the gel-forming layer 12a has the following features.

More specifically, a water absorption speed per 1g of the gel-forming layer 12a with respect to the water of 37°C is equal to or higher than 0.5 g/(g·s). By sufficiently increasing the water absorption speed as noted just above, the gel-forming layer 12a can rapidly absorb moisture from saliva within an oral cavity when the orally-administered agent 1 is administered to a patient, thereby forming a gel. In addition, the sufficiently high water absorption speed enables the gel-forming layer 12a to reliably absorb the moisture from the saliva and to form a gel even when the saliva exists in a small quantity within the oral cavity. As a result, the orally-administered agent 1 becomes soft.

When the gel-forming layer 12a is dipped into an aqueous solution of 0.9 mass% of sodium chloride (or physiological saline) having a temperature of 37°C, a gel fraction thereof is equal to or greater than 20 mass%. By sufficiently increasing the gel fraction as noted just above, the gel-forming layer 12a absorbing the moisture can maintain a gelatinized shape when the orally-administered agent 1 is administered to the patient. This enables the gel-forming layer 12a to be reliably provided on the surface of the orally-administered agent 1. In other words, it is possible to prevent the gel-forming layer 12a existing on the surface of the orally-administered agent 1 from being partially dissolved and to prevent the gel-forming layer 12a remaining in the orally-administered agent 1 from becoming thin. Accordingly, it is possible to prevent the medicine existing in the medicine-containing layer 11 from being dissolved and flowing out into the oral cavity. This makes it possible to mask a bitter taste or an offensive odor of the medicine.

As set forth above, the gel-forming layer 12a of the orally-administered agent 1 has an increased water absorption speed and a sufficiently high gel fraction. Therefore, the gel-forming layer 12a can form a gel by rapidly absorbing the moisture from the saliva within the oral cavity. The gel-forming layer 12a thus gelatinized is not dissolved within the oral cavity but is kept in a gel state. Consequently, the orally-administered agent 1 becomes soft in a rapid manner so that it can be swallowed with ease.

In contrast, if the water absorption speed of the gel-forming layer 12a is lower than the lower limit value stated above, the gel-forming layer 12a is not sufficiently and rapidly gelatinized, which makes it impossible to impart great enough softness to the orally-administered agent 1. As a result, it becomes difficult to swallow the orally-administered agent 1. In particular, an aged person or a patient suffering from a saliva secretion disorder may have a reduced quantity of saliva within an oral cavity and therefore may encounter a great difficulty in swallowing the orally-administered agent 1.

If the gel fraction of the gel-forming layer 12a available when dipped into the physiological saline is smaller than the lower limit value noted above, the gel-forming layer 12a becomes easily dissolvable, which in turn makes the orally-administered agent 1 easily dissolvable. Thus, the medicine flows out of the medicine-containing layer 11 when the orally-administered agent 1 is administered to a patient. In this case, the patient feels the bitter taste of the medicine and has a difficulty in swallowing the orally-administered agent 1. Depending on a kind of the medicines used, therefore, it becomes difficult to transport a target medicinal agent to a target location within a body using the orally-administered agent 1.

The water absorption speed of the gel-forming layer 12a is preferably in the range of 0.60 to 1.5 g/(g·s) and more preferably in the range of 0.70 to 1.0 g/(g-s), although it may vary within the range noted above. This makes it possible to further enhance the advantageous effects set forth above.

The gel fraction of the gel-forming layer 12a available when dipped into the physiological saline is preferably in the range of 25 to 70 mass% and more preferably in the range of 30 to 65 mass%, although it may vary within the range noted above. This makes it possible to impart great enough softness to the gel-forming layer 12a and hence the orally-administered agent 1, while surely preventing dissolution of the gel-forming layer 12a and maintaining the shape of the gel-forming layer 12a, when the gel-forming layer 12a absorbs the moisture.

The term "gel fraction" used herein means a value obtainable when a post-dipping solid content mass of a sample is divided by a pre-dipping solid content mass thereof. In this regard, the sample is dipped into an aqueous solution of 0.9 mass% of sodium chloride (or physiological saline) having a temperature of 37°C for 24 hours.

The term "water-based liquid" used herein denotes a liquid constituted of water and/or a liquid having increased compatibility to the water (e.g., liquid whose degree of solubility to 100 g of water at 25°C is 30 g or more). Although the water-based liquid is constituted of water and/or the liquid having increased compatibility to the water, it is preferred that the water-based liquid is mainly constituted of water. A content of water in the water-based liquid is preferably 90 mass% or more and more preferably 95 mass% or more. The saliva existing within the oral cavity falls under the category of the water-based liquid.

The gel-forming layer 12a contains a gel-forming agent that can be swelled and gelatinized by absorbing the water-based liquid. The gel-forming layer 12a containing the gel-forming agent can form a gel by easily and rapidly absorbing the water-based liquid existing therearound.

Examples of the gel-forming agent include, but are not limited to: a carboxyl group-containing polymer such as polyacrylic acid, a polyacrylic acid copolymer, polymethacrylic acid, a polymethacrylic acid copolymer, polyitaconic acid, a polyitaconic acid copolymer, a carboxy vinyl polymer, carboxymethyl cellulose, carboxymethyl hydroxyethyl cellulose, algin acid, heparin, hyaluronic acid, carrageenan, pectin acid, gelatin, collagen, gellan gum, casein and xanthane gum; starch and its derivatives; agar; arabinogalactan; galactomannan; dextran; and tragacanth. One or more of these substances can be used independently or in combination. The carboxyl group-containing polymer may contain a neutralized base obtained by neutralizing carboxyl groups with a monovalent base-forming cation. Examples of the base-forming cation include sodium and potassium.

It is preferred that the gel-forming agent includes the carboxyl group-containing polymer. The carboxyl group-containing polymer can rapidly absorb the water-based liquid and can form the gel in a rapid manner. In addition, the carboxyl group-containing polymer is a component relatively hard to dissolve after formation of the gel. Therefore, the gel-forming layer 12a is reliably kept in a gelatinized shape within the oral cavity. Use of polyacrylic acid as the carboxyl group-containing polymer makes it possible to further enhance the advantageous effects set forth above.

In the instance noted above, a viscosity at 20°C of an aqueous solution of 0.2 mass% of the carboxyl group-containing polymer is preferably in the range of 1500 to 50000 mPa·s and more preferably in the range of 10000 to 20000 mPa·s. This enables the gel-forming layer 12a to rapidly absorb the water-based liquid and to form the gel in the rapid manner. The gel-forming layer 12a is reliably kept in the gelatinized shape.

In case where the carboxyl group-containing polymer is used as the gel-forming agent, it may be possible to cross-link the carboxyl group-containing polymer through the use of a cross-linking agent. This ensures that the gelatinized gel-forming layer 12a shows increased gel strength in a swelling process and is surely prevented from dissolution.

The cross-linking can be performed by the cross-linking agent that varies with a kind of molecules to be cross-linked. As the cross-linking agent for the carboxyl group-containing polymer, it is possible to use, e.g., a polyvalent metal compound. Use of the polyvalent metal compound for the cross-linking purpose is preferable in that it is possible to reduce adherence of the orally-administered agent 1 to a mucosal membrane of the oral cavity in the administration process and to improve a shape-keeping ability of the gel in the swelling process.

Examples of the polyvalent metal compound include, but are not limited to, calcium chloride, magnesium chloride, aluminum chloride, aluminum sulfate, aluminum potassium sulfate, ferric chloride alum, ammonium alum, ferric sulfate, aluminum hydroxide, aluminum silicate, aluminum phosphate, iron citrate, magnesium oxide, calcium oxide, zinc oxide and zinc sulfate. One or more of these compounds can be used independently or in combination. Use of a trivalent metal compound among these compounds makes it possible to surely prevent dissolution of the carboxyl group-containing polymer, while increasing a cross-linking degree of the carboxyl group-containing polymer and enhancing physical strength of the gel-forming layer 12a.

A content of the gel-forming agent in the gel-forming layer 12a is preferably in the range of 5 to 90 mass% and more preferably in the range of 15 to 70 mass%, although it can be suitably adjusted depending on a kind of gel-forming agent or other factors. This enables the gel-forming layer 12a to rapidly absorb the water-based liquid, while surely preventing the gel-forming layer 12a from being dissolved after gelatinization.

In case where the cross-linking agent is contained in the gel-forming layer 12a, a content of the cross-linking agent in the gel-forming layer 12a is preferably in the range of 0.1 to 2.5 mass% and more preferably in the range of 0.5 to 1.2 mass%. This makes it possible to surely prevent dissolution of the gel-forming layer 12a while easily keeping the gel-forming layer 12a in the gelatinized shape. In addition, it is possible to reduce a viscosity of a coating solution used as a raw material of the gel-forming layer 12a in the below-mentioned process of producing the orally-administered agent 1, which makes it possible to efficiently form the gel-forming layer 12a.

The gel-forming layer 12a may contain a base (namely, a gel-forming base agent) which is a component contributing to stabilization of the shape of the gel-forming layer 12a. In other words, the base imparts a suitable degree of flexibility to the gel-forming layer 12a before the latter is swelled by the water-based liquid, thereby preventing the orally-administered agent 1 from being cracked or damaged by an external force or other causes. After the gel-forming layer 12a has absorbed the water-based liquid, the base serves to reliably keep the gel-forming layer 12a in the gelatinized shape, which prevents the gel from flowing out.

Examples of the base used in the gel-forming layer 12a include, but are not limited to, polyvinyl alcohol, polyvinyl pyrolidone, polyvinyl acetate, polyvinylphthalate acetate, hydroxyalkyl cellulose (e.g., hydroxypropyl cellulose, hydroxypropylmethyl cellulose, hydroxymethyl cellulose or hydroxyethyl cellulose), and alkyl cellulose (e.g., methyl cellulose or ethyl cellulose), one or more of which can be used independently or in combination.

In case where the base is contained in the gel-forming layer 12a, a content of the base in the gel-forming layer 12a is preferably in the range of 20 to 85 mass% and more preferably in the range of 30 to 80 mass%.

It is preferred that the base contained in the gel-forming layer 12a is water-soluble. If this is the case, it becomes easy for the moisture to get into the gel-forming layer 12a, thereby enabling the gel-forming layer 12a to be rapidly swelled and gelatinized within the oral cavity.

Examples of the water-soluble base include: polyvinyl alcohol; hydroxyalkyl cellulose such as hydroxypropyl cellulose, hydroxypropylmethyl cellulose, methyl cellulose or the like; and polyvinyl pyrolidone.

In particular, if polyvinyl alcohol is used as the base contained in the gel-forming layer 12a, it can mask the taste or order of the medicine contained in the medicine-containing layer 11. That is to say, polyvinyl alcohol serves as a masking agent to be described later.

The gel-forming layer 12a may contain a water absorption promoter for promoting water absorption of the gel-forming layer 12a. If this is the case, it becomes possible to sufficiently increase the water absorption speed of the gel-forming layer 12a within the oral cavity.

As the water absorption promoter, it is possible to use, e.g., a component having relatively high water-solubility. This component is dissolved in water and therefore can transport water into the gel-forming layer 12a.

A viscosity at 37°C of an aqueous solution of 5 mass% of the water absorption promoter is preferably in the range of 0.3 to 5.0 mPa·s, more preferably in the range of 0.5 to 3.5 mPa·s and even more preferably in the range of 0.6 to 1.8 mPa·s. As an indicator of water solubility of the water absorption promoter, it is possible to use the viscosity of the aqueous solution in which the water absorption promoter is dissolved. It is possible to think that the water solubility of the water absorption promoter is reduced as the viscosity of the aqueous solution grows low. If the viscosity of the aqueous solution of 5 mass% of the water absorption promoter falls within the range noted above, the water absorption promoter has a sufficiently high degree of water solubility within the oral cavity. This makes it possible to sufficiently increase the water absorption speed of the gel-forming layer 12a and to surely prevent the water absorption promoter from being suddenly dissolved and dispersed into the saliva.

Examples of the water absorption promoter include, but are not limited to: glycols such as propylene glycol, polyethylene glycol, polypropylene glycol, polyoxyl stearate, polyoxyethylene polyoxypropylene glycol, polyoxyethylene-cured castor oil and the like; glycerin; and sugars such as erythritol, sorbitol, xylitol, mannitol, inositol, maltitol, lactitol, glucose, xylose, mannose, fructose, galactose, sucrose, fructose, saccharose and the like, one or more of which can be used independently or in combination.

It is preferred that the water absorption promoter contains glycerin among the compounds listed above. Glycerin is a component that has increased capability to promote the water absorption of the gel-forming layer 12a while imparting softness to the gel-forming layer 12a. Therefore, the orally-administered agent 1 has a suitable degree of flexibility until it is administered to a patient, which means that the orally-administered agent 1 is hardly broken or damaged by an external force. When the orally-administered agent 1 is administered to the patient, the gel-forming layer 12a becomes soft within the oral cavity while keeping its shape unchanged. Thus, the orally-administered agent 1 becomes easy to swallow. In addition, glycerin tastes sweet and has an ability to mask the bitter taste or odor of the medicine within the oral cavity.

A content of glycerin in the water absorption promoter is preferably in the range of 35 to 95 mass% and more preferably in the range of 40 to 90 mass%. This ensures that the orally-administered agent 1 becomes soft and easy to swallow, while increasing the water absorption speed of the gel-forming layer 12a.

It is preferred that the water absorption promoter contains a solid-state compound in an atmosphere of 1 atm at 25°C. Although the water absorption promoter is a component having relatively high water solubility, the addition of the solid-state compound makes it possible to prevent the orally-administered agent 1 from absorbing moisture during storage thereof. Therefore, it is possible to surely prevent the orally-administered agent 1 from being degraded during the storage and to prevent the gel-forming layer 12a from being inadvertently gelatinized. In case where glycerin is contained in the water absorption promoter, the solid-state compound can prevent glycerin from bleeding out during the storage of the orally-administered agent 1.

Examples of the solid-state compound include the glycols stated above and the afore-mentioned sugars excepting glycerin.

If the water absorption promoter contains sugars, it becomes possible to attain the following advantageous effects. More specifically, sugars can serve as a masking agent to be described later, because they taste sweet and have increased capability to promote the water absorption of the gel-forming layer 12a. The sweet taste felt by a patient helps accelerate secretion of the saliva. As a result, the orally-administered agent 1 shows increased swallowability. Sugars and glycerin are similar in a chemical structure thereof and have an extremely high affinity with respect to each other. Accordingly, sugars are capable of reliably holding glycerin in the gel-forming layer 12a and surely preventing glycerin from bleeding out from the orally-administered agent 1.

If the water absorption promoter contains glycols, it becomes possible to attain the following advantageous effects. Glycols show a good affinity to water and have a chain-like structure, which means that glycols can be easily intertwined to other molecules. Therefore, glycols are linked to other molecules in the gel-forming layer 12a, thus maintaining the shape of the gel-forming layer 12a. This ensures that the gel-forming layer 12a is gelatinized with great ease while maintaining its shape. As a result, the orally-administered agent 1 shows especially high swallowability.

It is preferred that the glycols contain a compound having a structure of ethylene glycol chains. This makes it possible to surely prevent the glycols from bleeding out from the gel-forming layer 12a. Furthermore, this greatly increases the water absorption speed of the gel-forming layer 12a, which ensures that the orally-administered agent 1 can be swallowed in a rapid manner.

A hydrophile-lipophile balance (HLB) of the glycols is preferably in the range of 15 to 20 and more preferably in the range of 17 to 20. This makes it possible to surely prevent dissolution of the gel-forming layer 12a within the oral cavity while greatly increasing the water absorption speed thereof.

The hydrophile-lipophile balance (HLB) can be calculated by, e.g., a Kawakami method.

A mass-average molecular weight of the glycols is preferably in the range of 600 to 35000 and more preferably in the range of 1500 to 20000. This makes it possible to surely prevent the glycols from bleeding out from the gel-forming layer 12a. Furthermore, this greatly increases the water absorption speed of the gel-forming layer 12a, which ensures that the orally-administered agent 1 can be swallowed in the rapid manner.

It is preferred that the water absorption promoter contains, as the glycols, at least one of polyoxyl stearate (40) and polyoxyethylene (105) polyoxypropylene (5) glycol. This makes it possible to attain the following advantageous effects. More specifically, these components serve to greatly promote the water absorption of the gel-forming layer 12a and serve as a surfactant. This makes it possible to form a clear boundary face at the interface (surface) of the gel-forming layer 12a with the outside thereof. Therefore, it becomes easy to maintain the shape of the gelatinized gel-forming layer 12a constant, while preventing breakdown of the gel-forming layer 12a. Consequently, the orally-administered agent 1 shows increased swallowability. In case where the water absorption promoter contains glycols and glycerin, the gel-forming layer 12a exhibits increased water absorption speed. Therefore, there is also provided an advantageous effect of preventing the orally-administered agent 1 from adhering to an oral-cavity mucosal membrane, a esophagus, a gastric wall, and so forth. In addition, the addition of such glycols to the water absorption promoter makes it possible to reduce a viscosity of a coating solution of the gel-forming layer 12a in the below-mentioned process of producing the orally-administered agent 1. As a result, it is possible to evenly form the gel-forming layer 12a with a uniform thickness. It is also possible to increase a solid content concentration of the coating solution and to save the drying time and energy in the process of forming the gel-forming layer 12a.

It is desirable to satisfy preferably the relation of 0.05≤B/A≤1.5 and more preferably the relation of 0.10≤B/A≤1.0, where A denotes the content (mass%) of glycerin in the gel-forming layer 12a and B signifies the content (mass%) of the solid-state compound in the atmosphere of 1 atm at 25°C. This makes it possible to surely prevent the gel-forming layer 12a from being dissolved and bleeding out into the oral cavity, while greatly enhancing the softness of the orally-administered agent 1.

A content of the water absorption promoter in the gel-forming layer 12a is preferably in the range of 1 to 20 mass% and more preferably in the range of 3 to 17 mass%. This makes it possible to greatly increase the water absorption speed of the gel-forming layer 12a, while keeping the gel-forming layer 12a in a desired gel shape within the oral cavity.

The gel-forming layer 12a may contain a plasticizer. This imparts a proper degree of the softness to the gel-forming layer 12a. Examples of the plasticizer include glycerin triacetate, diethyl phthalate, triethyl citrate and laurylic acid, one or more of which can be used independently or in combination.

The gel-forming layer 12a may contain a masking agent capable of masking the taste or odor of the medicine contained in the medicine-containing layer 11. This makes it possible to enhance the effect of masking the taste or odor of the medicine (namely, what is called a masking effect) provided by the gel-forming layer 12a. Examples of the masking agent include: acidic-taste imparting agents such as citric acid, tartaric acid, fumaric acid and the like; sweetening agents such as saccharin, glycyrrhizinic acid and the like; mouth fresheners such as menthol, mentha oil, peppermint, spearmint and the like; and natural or synthetic perfumes, one or more of which can be used independently or in combination. The afore-mentioned sugars as the water absorption promoter have a sweet taste and can serve as the masking agent.

The gel-forming layer 12a may contain other components than mentioned above. For example, the gel-forming layer 12a may contain: antiseptic agents such as methyl hydroxybezoate, propyl hydroxybezoate and the like; and coloring agents such as edible lake pigment and the like.

A thickness of the gel-forming layer 12a is preferably in the range of 10 to 1000 µm and more preferably in the range of 15 to 500 µm, although it may be suitably adjusted within an orally-administrable range. If the thickness of the gel-forming layer 12a is smaller than 10 µm, the gel-forming layer 12a is gelatinized insufficiently and the effect of masking the taste or odor of the medicine provided by the gel-forming layer 12a becomes insufficient. In contrast, if the thickness of the gel-forming layer 12a is greater than 1000 µm, the gel-forming layer 12a cannot be sufficiently swelled and gelatinized only with the saliva when the orally-administered agent 1 is administered into the oral cavity of the patient. This makes it difficult for a patient to take the orally-administered agent 1.

The orally-administered agent 1 can be produced by, e.g., the following process.

### (Gel-forming Layer production Step)

Prepared first is a coating solution (namely, a coating solution for the gel-forming layer) containing constituent materials of the gel-forming layer.

The coating solution for the gel-forming layer can be prepared by dispersing or dissolving the constituent materials of the gel-forming layer as described above in a liquid medium such as purified water, ethanol or the like.

Next, the coating solution for the gel-forming layer is applied or sprayed on a supporting substrate and then dried. This produces a gel-forming layer.

As the supporting substrate, it is possible to use, e.g., a glass plate, a plastic film or a release sheet, but is not limited to them.

### (Intermediate Body Production Step)

Prepared next is a coating solution (namely, a coating solution for the medicine-containing layer) containing constituent materials of the medicine-containing layer.

The coating solution for the medicine-containing layer can be prepared by dispersing or dissolving the constituent materials of the medicine-containing layer as described above in a liquid medium such as purified water, ethanol or the like.

Next, the coating solution for the medicine-containing layer is applied or sprayed on the gel-forming layer of the supporting substrate and then dried. This produces a precursor of the medicine-containing layer (namely, a precursor for the medicine-containing layer) and eventually produces an intermediate body for the orally-administered agent (hereinafter simply referred to as an intermediate body) consisting of the precursor for the medicine-containing layer and the gel-forming layer.

### (Thermal Compression Bonding Step)

Next, two sheets of the intermediate body produced in the preceding step are prepared and thermally fusion-bonded together under a pressure so that the precursors of the two medicine-containing layers of the intermediate bodies can be bonded to each other. Thus, the precursors of the two medicine-containing layers are fusion-bonded to form a single medicine-containing layer. This produces a laminated body consisting of the two gel-forming layers 12a and 12b, and the medicine-containing layer. The laminated body may be used as the orally-administered agent as it stands, or may be punched into an arbitrary shape, such as a circular shape, an elliptical shape or a polygonal shape, to produce the orally-administered agent.

In case where the medicine-containing layers are thermally fusion-bonded together through the thermal compression bonding step, it is preferred that the medicine-containing layers contain, as the base (namely, the base agent for the medicine-containing layer), thermally fusion-bondable polymer such as polyvinyl acetate, polyvinyl pyrolidone, vinyl acetate-vinyl pyrolidone copolymer or the like.

The orally-administered agent 1 may be produced by, e.g., repeating the tasks of applying and drying the coating solution for the gel-forming layer and the coating solution for the medicine-containing layer.

### <Second Embodiment>

FIG. 2 is a section view showing an orally-administered agent in accordance with a second embodiment of the present invention.

The second embodiment of the present invention will now be described with reference to FIG. 2. The following description will be centered on the points differing from the first embodiment, with the same items omitted from the description.

As shown in FIG. 2, the orally-administered agent 1a of the present embodiment differs from that of the first embodiment in that major surfaces of gel-forming layers 12c and 12d defining outer surfaces of the orally-administered agent 1a have a plurality of convex portions 121.

Provision of the convex portions 121 on the outer surfaces of the gel-forming layers 12c and 12d makes it possible to greatly increase a speed at which the gel-forming layers 12c and 12d absorb the water-based liquid from the saliva within the oral cavity. In other words, a contact area between the saliva and each of the gel-forming layers 12c and 12d can be increased by forming the convex portions 121, which results in a sharp increase in the water absorption speed of the gel-forming layers 12c and 12d. In addition, a contact area between the orally-administered agent 1a and the inner wall of the oral cavity can be reduced by forming the convex portions 121 on the outer surfaces of the gel-forming layers 12c and 12d. This surely prevents the orally-administered agent 1a from adhering to the inner wall of the oral cavity. Thanks to the features noted above, the orally-administered agent 1a exhibits especially high swallowability.

The pitch p between the convex portions 121 of the gel-forming layers 12c and 12d is not particularly limited but may be preferably 100 to 1000 µm and more preferably 250 to 750 µm. This surely prevents the orally-administered agent 1a from adhering to the inner wall of the oral cavity, while greatly increasing the water absorption speed.

The width w of each of the convex portions 121 of the gel-forming layers 12c and 12d is not particularly limited but may be preferably in the range of 20 to 300 µm and more preferably in the range of 50 to 250 µm. This surely prevents the orally-administered agent 1a from adhering to the inner wall of the oral cavity, while greatly increasing the water absorption speed. It is also possible to surely prevent the gel-forming layers 12c and 12d from being dissolved in the saliva.

The height d of each of the convex portions 121 of the gel-forming layers 12c and 12d is not particularly limited but may be preferably in the range of 10 to 5000 µm and more preferably in the range of 20 to 1000 µm. This surely prevents the orally-administered agent 1a from adhering to the inner wall of the oral cavity, while greatly increasing the water absorption speed. It is also possible to surely prevent the gel-forming layers 12c and 12d from being dissolved in the saliva.

Alternatively, a plurality of concave portions 122 of the gel-forming layers 12c and 12d may extend through the thickness of each of the gel-forming layers 12c and 12d or through the full thickness of the orally-administered agent 1a.

The gel-forming layers 12c and 12d having the convex portions 121 set forth above can be produced by, e.g., forming, on a surface of a supporting substrate, concave portions having a pattern complementary to that of the convex portions 121 to be formed, applying the coating solution containing the constituent materials of the gel-forming layers 12c and 12d on the supporting substrate and drying the coating solution, when the gel-forming layers 12c and 12d are produced. Alternatively, the gel-forming layers 12c and 12d having the convex portions 121 may be produced by, e.g., producing the orally-administered agent 1 in the same manner as used in the first embodiment and then pressing a substrate, which has concave portions of a pattern complementary to that of the convex portions to be formed, against the orally-administered agent 1 while heating the substrate and the orally-administered agent 1, if necessary.

While the illustrated embodiments of the present invention have been described hereinabove, the present invention shall not be limited thereto. In particular, the orally-administered agent according to the present invention is not limited to the one including the two gel-forming layers and the medicine-containing layer. For example, the orally-administered agent according to the present invention may be comprised of one gel-forming layer and the medicine-containing layer.

As an alternative example, the orally-administered agent according to the present invention may include a plurality of medicine-containing layers containing a medicine of the same kind or medicines of different kinds.

As a further alternative example, orally-administered agent according to the present invention may include additional arbitrary layers formed between each of the gel-forming layers and the medicine-containing layer. For example, the orally-administered agent may further include an adhesive layer for enhancing cohesion between each of the gel-forming layers and the medicine-containing layer or an elution-preventing layer for preventing elution of the medicine.

As a still further alternative example, it may be possible to provide an orally-administered agent 1b as shown in FIG. 3, in which a medicine-containing layer 11 is surrounded by gel-forming layers 12e and 12f. This makes it possible to surely prevent the medicine contained in the medicine-containing layer from being dissolved within the oral cavity.

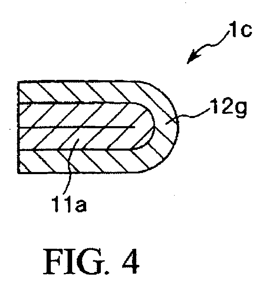

As a yet still further alternative example, it may be possible to provide an orally-administered agent 1c as shown in FIG. 4, which includes a medicine-containing layer 11a and a single gel-forming layer 12g. In this case, the medicine-containing layer 11a and the gel-forming layer 12g are folded so that the medicine-containing layer 11a can be surrounded by the gel-forming layer 12g.

### Examples

Next, certain production examples of the orally-administered agent according to the present invention will be described in detail.

### 1. Production of Orally-administered Agent (Example 1)

### (a) Gel-forming Layer Production Step

Coating solution A containing constituent materials of a gel-forming layer was prepared first.

1.5 mass parts of calcium chloride (defined in Japanese Pharmacopoeia and produced by Tomita Pharmaceutical Co., Ltd.) was added to 1050 mass parts of purified water. The resultant mixture was stirred sufficiently to dissolve calcium chloride. Then, 56.5 mass parts of polyacrylic acid (Carbopol 974P produced by CBC Co., Ltd., a viscosity of an aqueous solution of 0.2 mass% is 12100 mPa·s) was slowly added to the resultant mixture while stirring the same to obtain a mixture. Thereafter, the mixture was stirred for about one hour. Then, 33.9 mass parts of polyvinyl alcohol (Gosenol EG05 produced by Nippon Kasei Chemical Co., Ltd.) was slowly added to the mixture while stirring the same. Thereafter, the mixture to which the respective materials had added was heated to 70°C and stirred for about one hour. Subsequently, 8.1 mass parts of glycerin (thick glycerin defined in Japanese Pharmacopoeia and produced by ADEKA Corp.) as a water absorption promoter was added to the mixture and stirred for about ten minutes, thereby producing the coating solution A.

Next, the coating solution A was sufficiently defoamed and flat-applied on the opposite surface of a release treatment surface of a polyester terephthalate film (SP-PET3811 produced by Lintec Corp.) through the use of an applicator whose gap was set so that a post-drying application quantity of the coating solution A could become 20 g/m². The coating solution A thus applied was dried at 80°C for six minutes, thus producing a gel-forming layer. Some portions of the gel-forming layer thus produced were used in measuring the water absorption speed and the gel fraction to be described later.

### (b) Intermediate Body Production Step

Coating solution B containing constituent materials of a medicine-containing layer was prepared first.

2.5 mass parts of famotidine as a gastric ulcer medicine and 0.6 mass parts of titanium oxide (TIPAQUE CR-50 produced by Ishihara Sangyo Kaisha, Ltd.) were added to 53.7 mass parts of purified water and sufficiently dispersed through the use of a homogenizer. Thereafter, 13.8 mass parts of polyvinyl pyrolidone (PVP K-90 produced by ISP Japan Ltd.) was slowly added to the resultant mixture while stirring the same to obtain a mixture. Then, the mixture was stirred for about thirty minutes. Subsequently, 4.0 mass parts of glycerin (thick glycerin defined in Japanese Pharmacopoeia and produced by ADEKA Corp.) was added to the mixture and stirred for about five minutes, thereby producing the coating solution B.

Next, the coating solution B was sufficiently defoamed and flat-applied on the gel-forming layer produced in the step (a), through the use of an applicator whose gap was set so that a post-drying application quantity of the coating solution B could become 50 g/m². The coating solution B thus applied was dried at 80°C for five minutes, thus producing a laminated body (or an intermediate body) that consists of a medicine-containing layer precursor and the gel-forming layer.

### (c) Thermal Compression Bonding Step

Two sheets of the intermediate body produced in the step (b) were thermally fusion-bonded together at a temperature of 100°C, under a pressure of 1 kgf/cm² and for one second so that the medicine-containing layer precursors thereof could be bonded to each other. Then, the polyester terephthalate films were separated from the gel-forming layers, thereby producing a laminated body that consists of the gel-forming layer, a medicine-containing layer and the gel-forming layer. A circular film having a diameter of 15 mm was punched from the laminated body to obtain an orally-administered agent.

### (Examples 2 to 16)

Orally-administered agents were obtained in the same manner as in the Example 1, except that the kind and content of the respective constituent materials of the coating solution A were changed as shown in Table 1.

### (Example 17)

An orally-administered agent was obtained in the same manner as in the Example 1, except that the content of the respective constituent materials of the coating solution A used in the gel-forming layer production step was changed as shown in Table 1 and that the application conditions of the coating solution A (namely, the production conditions of the gel-forming layer) were changed as mentioned below.

The coating solution A was sufficiently defoamed and flat-applied on the opposite surface of a release treatment surface of a polyester terephthalate film (SP-PET3811 produced by Lintec Corp.) through the use of an applicator whose gap was set so that a post-drying application quantity of the coating solution A could become 20 g/m². The polyester terephthalate film had concave portions (having the mouth size of 450x450 µm, the depth of 30 µm and the bottom size of 184x184 µm) provided in a grid pattern at a pitch of 550 µm. The coating solution A thus applied was dried at 85°C for five minutes, thus producing a gel-forming layer. The gel-forming layer thus produced was provided with convex portions having the height of about 30 µm, the width of about 450 µm and the pitch of about 550 µm, the shape of which was transferred from the concave portions of the polyester terephthalate film.

### (Comparative Example)

An orally-administered agent was obtained in the same manner as in the Example 1, except that the kind and content of the respective constituent materials of the coating solution A were changed as shown in Table 1.

Table 1 shows the constituent materials of the gel-forming layer (coating solution A) and the content thereof in the respective Examples and the Comparative Example. In Table 1, the name "EG05" signifies polyvinyl alcohol (Gosenol EG05 produced by Nippon Kasei Chemical Co., Ltd.), the name "EG40" signifies polyvinyl alcohol (Gosenol EG40 produced by Nippon Kasei Chemical Co., Ltd.), the name "PEG" signifies polyethylene glycol #4000 (having the HLB of 20 and the molecular weight of 4000, which is produced by Kanto Chemical Co., Inc.), the name "S1" signifies polyoxyl stearate 40 (NIKKOL MYS-40MV having the HLB of 17.5 and the molecular weight of 2047, which is produced by Nikko Chemicals Co., Ltd.), the name "S2" signifies polyoxyethylene (105) polyoxypropylene (5) glycol (PEP-101 having the HLB of 20 and the molecular weight of 4000 to 5500, which is produced by Freund Corp.). In Table 1, the viscosity of the water absorption promoter denotes the viscosity at 37°C of an aqueous solution of 5 mass% of the water absorption promoter, which was measured with an E-type viscometer (a product of Tokimec, Inc.). In the Examples 1 to 4 and 17, the viscosity of the water absorption promoter denotes a viscosity of an aqueous solution of 5 mass% of glycerin. In the Examples 5 to 16 and the Comparative Example, the viscosity of the water absorption promoter denotes a viscosity of an aqueous solution of 5 mass% of other water absorption promoter than glycerin.

Table 1

**Table 1**

| | Base | | Gel-forming agent | | Cross-linking agent | | Water absorption promoter | | | | | B/A |
|---|---|---|---|---|---|---|---|---|---|---|---|---|
| | Kind | Content [Mass parts] | Kind | Content [Mass parts] | Kind | Content [Mass parts] | Kind | Content A [Mass parts] | Kind | Content B [Mass parts] | Viscosity [mPa · s] | |
| Ex. 1 | EG05 | 33.9 | Polyacrylic acid | 56.5 | Calcium chloride | 1.5 | Glycerin | 8.1 | - | - | 0.90 | - |
| Ex. 2 | EG05 | 68.5 | Polyacrylic acid | 22.8 | Calcium chloride | 0.6 | Glycerin | 8.1 | - | - | 0.90 | - |
| Ex. 3 | EG40 | 68.6 | Polyacrylic acid | 22.8 | Calcium chloride | 0.6 | Glycerin | 8.1 | - | - | 0.90 | - |
| Ex. 4 | EG40 | 80.2 | Polyacrylic acid | 11. 5 | Calcium chloride | 0.3 | Glycerin | 8.1 | - | - | 0.90 | - |
| Ex. 5 | EG05 | 68.2 | Polyacrylic acid | 22.7 | Calcium chloride | 0.6 | Glycerin | 8.1 | Mannitol | 0,5 | 0.87 | 0.06 |
| Ex. 6 | EG05 | 67.8 | Polyacrylic acid | 22.6 | Calcium chloride | 0.6 | Glycerin | 8.0 | Mannitol | 1.0 | 0.87 | 0. 12 |
| Ex. 7 | EG05 | 66.8 | Polyacrylic acid | 22.2 | Calcium chloride | 0.6 | Glycerin | 7.9 | Mannitol | 2.5 | 0.87 | 0.32 |
| Ex. 8 | EG05 | 65.8 | Polyacrylic acid | 21. 9 | Calcium chloride | 0.6 | Glycerin | 7.8 | Mannitol | 4.0 | 0.87 | 0.51 |
| Ex. 9 | EG05 | 65.1 | Polyacrylic acid | 21.7 | Calcium chloride | 0.6 | Glycerin | 7.7 | Mannitol | 5.0 | 0.87 | 0.65 |
| Ex. 10 | EG05 | 61.7 | Polyacrylic acid | 20.5 | Calcium chloride | 0.5 | Glycerin | 7.3 | Mannitol | 10.0 | 0.87 | 1.37 |
| Ex. 11 | EG40 | 66.8 | Polyacrylic acid | 22. 2 | Calcium chloride | 0.6 | Glycerin | 7.9 | Mannitol | 2.5 | 0. 87 | 0.32 |
| Ex. 12 | EG05 | 66.8 | Polyacrylic acid | 22.2 | Calcium chloride | 0.6 | Glycerin | 7.9 | Inositol | 2.5 | 0.93 | 0.32 |
| Ex. 13 | EG05 | 66.8 | Polyacrylic acid | 22.2 | Calcium chloride | 0.6 | Clycerin | 7.9 | Xylitol | 2,5 | 1.08 | 0.32 |
| Ex.14 | EG05 | 66.8 | Polyacrylic acid | 22.2 | Calcium chloride | 0.6 | Glycerin | 7.9 | PEG | 2.5 | 1.29 | 0.32 |
| Ex.15 | EG05 | 66.8 | Polyacrylic acid | 22.2 | Calcium chloride | 0.6 | Glycerin | 7.9 | S1 | 2.5 | 1.29 | 0.32 |
| Ex.16 | EG05 | 66.8 | Polyacrylic acid | 22.2 | Calcium chloride | 0.6 | Glycerin | 1.9 | S2 | 2.6 | 1. 62 | 0.32 |
| Ex. 17 | EG05 | 68.5 | Polyacrylic acid | 22.8 | Calcium chloride | 0.6 | Glycerin | 8.1 | - | - | 0.90 | - |
| Comp. Ex. | EG05 | 64.8 | Polyacrylic acid | 18.2 | Calcium chloride | 0.5 | Glycerin | 6.5 | Mannitol | 20.0 | 0.87 | 3.09 |

### 2. Measurement of Water Absorption Speed

Two sheets having a size of 20 mm x 60 mm were cut out from each of the gel-forming layers produced in the respective Examples and the Comparative Example. A mass W1 (g) of the sheets was measured. These sheets were put into a stainless basket having a diameter of 22.2 mm and a depth of 27 mm and were dipped for five seconds in purified water of about 90 mL and 37°C contained within a 200 mL plastic vessel. Thereafter, the purified water adhering to the basket was wiped out to measure a mass W2 (g) of the basket holding the dipped sheets. In a blank test, the stainless basket holding no gel-forming layer were dipped in the purified water of 37°C for five seconds in the same manner as above. The purified water adhering to the basket was wiped out to measure a mass W0 (g) of the empty basket. The water absorption speed of the gel-forming layers were calculated from the measured values according to the following equation (I): water absorption speed (g/(g·s))={(W2-W1-W0)/W1}/5.

### 3. Measurement of Gel Fraction

Two sheets having a size of 10 mm x 10 mm were cut out from each of the gel-forming layers produced in the respective Examples and the Comparative Example. One of the sheets (sheet A) was dried at 105°C for four hours. Then, a water content in the sheet A was calculated by comparing a pre-drying mass of the sheet A with a post-drying mass thereof.

Next, a mass of the other sheet (sheet B) was calculated and a solid content quantity M (g) of the sheet B was calculated based on the water content of the sheet A.

Then, the sheet B was put into a nylon bag (of 120 meshes having a mass of N (g)) and dipped for twenty four hours in an aqueous solution of 0.9 mass% of sodium chloride (physiological saline) having a temperature of 37°C.

The nylon bag and the sheet B taken out from the physiological saline after dipping were dried for four hours at a temperature of 105°C. Then, a mass O (g) of the nylon bag including the sheet B was measured.

A gel fraction of the gel-forming layers was calculated from the measured values according to the following equation (II): gel fraction (mass %)={(O-N)/M}.

### 4. Evaluation of swallowability (administrability)

Gargling was conducted to cleanse the interior of the oral cavity. After two minutes, each of the orally-administered agents produced in the respective Examples and the Comparative Example was put into the oral cavity to measure a time required in rendering the orally-administered agent to become swallowable. This measurement was performed three times and an average value was defined as a swallowable time.

### 5. Evaluation of Masking Ability

Gargling was conducted to cleanse the interior of the oral cavity. After two minutes, each of the orally-administered agents produced in the respective Examples and the Comparative Example was put into the oral cavity. After thirty minutes, the orally-administered agent was ejected from the oral cavity. The bitter taste felt while the orally-administered agent stays within the oral cavity was evaluated according to the following three criteria. This evaluation was performed ten times and an average value was calculated for the purpose of an overall evaluation.

3: The bitter taste of the medicine is seldom felt.

2: A certain degree of bitter taste of the medicine is felt but negligible.

1: The bitter taste of the medicine is felt strong and unpleasant.

The results of the evaluation are shown in Table 2.

Table 2

**Table 2**

| | Water absorption speed [/(g · s)] | Gel fraction [mass %] | Evaluation of swallowability [s] | Evaluation of masking ability |
|---|---|---|---|---|
| Ex. 1 | 0.63 | 62. 1 | 20.8 | 3.0 |
| Ex. 2 | 0.65 | 49.9 | 20.3 | 3.0 |
| Ex. 3 | 0.58 | 50.4 | 21.4 | 3.0 |
| Ex. 4 | 0.83 | 30.1 | 15.2 | 2.6 |
| Ex. 5 | 0.66 | 48.8 | 20.5 | 3.0 |
| Ex. 6 | 0.81 | 43.5 | 16.1 | 3.0 |
| Ex. 7 | 0.90 | 40.5 | 13.7 | 3.0 |
| Ex. 8 | 0.91 | 35.2 | 13.4 | 2.8 |
| Ex. 9 | 0.91 | 29.4 | 13.1 | 2.4 |
| Ex. 10 | 0.66 | 22.9 | 19.8 | 2.1 |
| Ex. 11 | 0.91 | 42.4 | 13.9 | 3.0 |
| Ex. 12 | 0.87 | 39.2 | 15.3 | 3.0 |
| Ex. 13 | 0.92 | 40.1 | 12.8 | 3.0 |
| Ex. 14 | 0.99 | 40.3 | 11.4 | 3.0 |
| Ex. 15 | 0.88 | 35.2 | 14.9 | 2.9 |
| Ex. 16 | 1.06 | 40.2 | 10.5 | 3.0 |
| Ex. 17 | 0.70 | 49.8 | 19.4 | 3.0 |
| Comp. Ex. | 0.29 | 12.3 | - | 1.3 |

As shown in Table 2, the orally-administered agents of the respective Examples exhibited good swallowability and could be swallowed within a relatively short period of time. In addition, the orally-administered agents of the respective Examples showed good masking ability and made it hard for a patient to feel the bitter taste of the medicine when swallowed the orally-administered agents. In contrast, no satisfactory results were obtained in the Comparative Example. In other words, the orally-administered agent of the Comparative Example was dissolved within the oral cavity and could not be swallowed without feeling the bitter taste of the medicine.

All water absorption promoters used in the respective Examples, except glycerin, were kept solid in an atmosphere of 1 atm at 25°C.

In each of the Examples 5 to 17 and the Comparative Example, the viscosity at 37°C of the aqueous solution of 5 mass% of the water absorption promoter was in the range of from 0.5 to 3.5 mPa·s.

In approval criteria of foods for aged persons (issued on February 23, 1994 as EISHIN No. 15), the Ministry of Health, Labor and Welfare of Japan stipulates that the hardness of the foods for a person who suffers from chewing or swallowing disorders should be 500 N/m² or less as a standard "may not bite". All gel-forming layers of the respective Examples showed hardness of 500 N/m² or less when fifteen seconds has lapsed after they were dipped into the water of 37°C.

### INDUSTRIAL APPLICABILITY

According to the present invention, it is possible to provide an orally-administered agent that can be rapidly gelatinized with a small quantity of water and can be swallowed with ease. Accordingly, the orally-administered agent according to the present invention has industrial applicability.

## Claims

1. An orally-administered agent comprised of a laminated body having surface layers, the laminated body comprising:
a medicine-containing layer containing a medicine and having surfaces; and
at least one gel-forming layer provided on one of the surfaces of the medicine-containing layer as one of the surface layers of the laminated body, wherein the at least one gel-forming layer includes a gel-forming agent to be swelled and gelatinized when absorbing a water-based liquid;
wherein a water absorption speed per 1 g of the gel-forming layer with respect to water of 37°C is 0.5 g/(g·s) or higher, and a gel fraction of the gel-forming layer is 20 mass% or more when the gel-forming layer is dipped into an aqueous solution of 0.9 mass% of sodium chloride having a temperature of 37°C,
wherein the gel-forming agent includes a carboxyl group-containing polymer, and
wherein the gel-forming layer includes a water absorption promoter for promoting absorption of the water-based liquid into the gel-forming layer, the water absorption promoter includes glycerin and at least one of a sugar or a glycol.

2. The orally-administered agent as claimed in claim 1, wherein the carboxyl group-containing polymer has a property that a viscosity of an aqueous solution of 0.2 mass% is in the range of 1500 to 50000 mPa·s at 20°C.

3. The orally-administered agent as claimed in claim 1, wherein a content of the water absorption promoter included in the gel-forming layer is in the range of 1 to 20 mass%.

4. The orally-administered agent as claimed in claim 1, wherein the water absorption promoter has a property that a viscosity of an aqueous solution of 5 mass% is in the range of 0.3 to 5.0 mPa·s at 37°C.

5. The orally-administered agent as claimed in claim 1, wherein a content of the glycerin included in the water absorption promoter is in the range of 35 to 95 mass%.

6. The orally-administered agent as claimed in claim 1, wherein the water absorption promoter includes a solid-state compound in an atmosphere of 1 atm at 25°C.

7. The orally-administered agent as claimed in claim 1, wherein the glycol includes a compound having an ethylene glycol chain in a chemical structure thereof.

8. The orally-administered agent as claimed in claim 1, wherein the glycol includes a surfactant of which hydrophile-lipophile balance (HLB) is in the range of 15 to 20.

9. The orally-administered agent as claimed in claim 1, wherein a mass-average molecular weight of the glycol is in the range of 600 to 35000.

10. The orally-administered agent as claimed in claim 1, wherein the gel-forming layer has an outer surface, and the outer surface has convex portions.

11. The orally-administered agent as claimed in claim 1, wherein the laminated body is formed into a film shape.

12. The orally-administered agent as claimed in claim 1, wherein the carboxyl group-containing polymer is cross-linked by a cross-linking agent.

13. The orally-administered agent as claimed in claim 12, wherein the cross-linking agent is a polyvalent metal compound.

14. The orally-administered agent as claimed in claim 1, wherein the carboxyl group-containing polymer is polyacrylic acid.

15. The orally-administered agent as claimed in claim 6, wherein a content of the glycerin in the gel-forming layer is defined as A (mass%) and a content of the solid-state compound is defined as B (mass%), and a relation 0.05≤B/A≤1.5 is satisfied.

16. The orally-administered agent as claimed in claim 1, wherein the water absorption promoter includes at least one of polyoxyl stearate 40 or polyoxyethylene (105) polyoxypropylene (5) glycol as the glycol.

## Patentansprüche

1. Oral verabreichtes Mittel, bestehend aus einem laminierten Körper mit Oberflächenschichten, wobei der laminierte Körper folgendes umfasst:
eine Medikament-enthaltende Schicht, enthaltend eine Medikament und mit Oberflächen; und
mindestens eine gelbildende Schicht, die auf einer der Oberflächen der Medikamententhaltenden Schicht als eine der Oberflächenschichten des laminierten Körperes vorgesehen ist, wobei die mindestens eine gelbildende Schicht ein bei Absorption einer wasserbasierten Flüssigkeit zu quellendes und zu gelierendes gelbildendes Mittel umfasst,
wobei eine Wasser-Absorptionsgeschwindigkeit pro 1 g der gelbildenden Schicht bezüglich Wasser von 37°C 0,5 g/(g · s) oder mehr beträgt, und eine Gelfraktion der gelbildenden Schicht 20 Masse-% oder mehr beträgt, wenn die gelbildende Schicht in eine wässrige Lösung von 0,9 Masse-% Natriumchlorid mit einer Temperatur von 37 °C eingetaucht wird,
wobei das gelbildende Mittel ein Carboxylgruppe-enthaltendes Polymer umfasst, und
wobei die gelbildende Schicht einen Wasserabsorptionsbeschleuniger zur Beschleunigung der Absorption der wasserbasierten Flüssigkeit in die gelbildende Schicht umfasst, wobei der Wasserabsorptionsbeschleuniger Glycerin und mindestens eines von einem Zucker oder einem Glycol umfasst.

2. Oral verabreichtes Mittel nach Anspruch 1, wobei das Carboxylgruppe-enthaltende Polymer eine Eigenschaft aufweist, dass eine Viskosität einer wässrigen Lösung im Bereich von 1500 bis 50.000 mPa.s bei 20 °C liegt.

3. Oral verabreichtes Mittel nach Anspruch 1, wobei ein Gehalt des Wasserabsorptionsbeschleunigers, der in der gelbildenden Schicht eingeschlossen ist, im Bereich von 1 bis 20 Masse-% liegt.

4. Oral verabreichtes Mittel nach Anspruch 1, wobei der Wasserabsorptionsbeschleuniger eine Eigenschaft aufweist, dass eine Viskosität einer wässrigen Lösung von 5 Masse-% im Bereich von 0,3 bis 5,0 mPa bei 37°C liegt.

5. Oral verabreichtes Mittel nach Anspruch 1, wobei ein Gehalt des in den Wasserabsorptionsbeschleuniger eingeschlossenen Glycerins im Bereich von 35 bis 95 Masse-% liegt.

6. Oral verabreichtes Mittel nach Anspruch 1, wobei der Wasserabsorptionsbeschleuniger eine Festkörperverbindung in einer Atmosphäre von 1 atm bei 25 °C aufweist.

7. Oral verabreichtes Mittel nach Anspruch 1, wobei das Glycol eine Verbindung mit einer Ethylenglycolkette in einer chemischen Struktur davon einschließt.

8. Oral verabreichtes Mittel nach Anspruch 1, wobei das Glycol ein grenzflächenaktives Mittel umfasst, dessen Hydrophilie-Lipophilie-Gleichgewicht (HLB) im Bereich von 15 bis 20 liegt.

9. Oral verabreichtes Mittel nach Anspruch 1, wobei ein massegemitteltes Molekulargewicht des Glycols im Bereich von 600 bis 35.000 liegt.

10. Oral verabreichtes Mittel nach Anspruch 1, wobei die gelbildende Schicht eine äußere Oberfläche aufweist und die äußere Oberfläche konvexe Teile aufweist.

11. Oral verabreichtes Mittel nach Anspruch 1, wobei der laminierte Körper zu einer Filmform geformt ist.

12. Oral verabreichtes Mittel nach Anspruch 1, wobei das Carboxylgruppe-enthaltende Polymer durch ein Vernetzungsmittel vernetzt ist.

13. Oral verabreichtes Mittel nach Anspruch 12, wobei das Vernetzungsmittel eine mehrwertige Metallverbindung ist.

14. Oral verabreichtes Mittel nach Anspruch 1, wobei das Carboxylgruppe-enthaltende Polymer Polyacrylsäure ist.

15. Oral verabreichtes Mittel nach Anspruch 6, wobei ein Gehalt des Glycerins in der gelbildenden Schicht als A (Masse-%) definiert ist und ein Gehalt der Festkörperverbindung als B (Masse-%) definiert ist und eine Beziehung 0,05≤B/A≤1,5 erfüllt ist.

16. Oral verabreichtes Mittel nach Anspruch 1, wobei der Wasserabsorptionsbeschleuniger mindestens ein Polyoxylstearat 40 oder Polyoxyethylen (105)-Polyoxypropylen (5)-Glycol als das Glycol umfasst.

## Revendications

1. Agent à administrer par voie orale composé d'un corps stratifié présentant des couches de surface, le corps stratifié comprenant :
une couche contenant un médicament, ladite couche contenant un médicament et présentant des surfaces ; et
au moins une couche formant un gel prévue sur une des surfaces de la couche contenant un médicament en tant qu'une des couches de surface du corps stratifié, dans lequel l'au moins une couche formant un gel comprend un agent formant un gel qui se gonfle et se gélatinise lors de l'absorption d'un liquide à base d'eau ;
dans lequel la vitesse d'absorption de l'eau pour 1 g de couche formant un gel pour de l'eau à 37 °C est supérieure ou égale à 0,5 g/(g · s), et une fraction gel de la couche formant un gel est supérieure ou égale à 20 % en poids lorsque la couche formant un gel est trempée dans une solution aqueuse à 0,9 % en poids de chlorure de sodium ayant une température de 37 °C,
dans lequel l'agent formant un gel comprend un polymère contenant un groupe carboxyle, et
dans lequel la couche formant un gel comprend un promoteur de l'absorption d'eau destiné à favoriser l'absorption du liquide à base d'eau à l'intérieur de la couche formant un gel, le promoteur de l'absorption d'eau comprend de la glycérine et au moins un composé parmi un sucre ou un glycol.

2. Agent à administrer par voie orale selon la revendication 1, dans lequel le polymère contenant un groupe carboxyle est **caractérisé par le fait que** la viscosité d'une solution aqueuse à 0,2 % en poids se situe dans la plage allant de 1 500 à 50 000 mPa s à 20 °C.

3. Agent à administrer par voie orale selon la revendication 1, dans lequel une teneur du promoteur de l'absorption d'eau inclus dans la couche formant un gel se situe dans la plage allant de 1 à 20 % en poids.

4. Agent à administrer par voie orale selon la revendication 1, dans lequel le promoteur de l'absorption d'eau est **caractérisé par le fait que** la viscosité d'une solution aqueuse à 5 % en poids se situe dans la plage allant de 0,3 à 5,0 mPa s à 37 °C.

5. Agent à administrer par voie orale selon la revendication 1, dans lequel une teneur de la glycérine incluse dans le promoteur de l'absorption d'eau se situe dans la plage allant de 35 à 95 % en poids.

6. Agent à administrer par voie orale selon la revendication 1, dans lequel le promoteur de l'absorption d'eau comprend un composé à l'état solide sous une atmosphère de 1 atm et à 25 °C.

7. Agent à administrer par voie orale selon la revendication 1, dans lequel le glycol comprend un composé présentant une chaîne éthylène glycol dans une structure chimique de celui-ci.

8. Agent à administrer par voie orale selon la revendication 1, dans lequel le glycol comprend un tensio-actif dont la balance hydrophile-lipophile (HLB) se situe dans la plage allant de 15 à 20.

9. Agent à administrer par voie orale selon la revendication 1, dans lequel un poids moléculaire moyen en poids du glycol se situe dans la plage allant de 600 à 35 000.

10. Agent à administrer par voie orale selon la revendication 1, dans lequel la couche formant un gel présente une surface externe, et la surface externe présente des parties convexes.

11. Agent à administrer par voie orale selon la revendication 1, dans lequel le corps stratifié a la forme d'un film.

12. Agent à administrer par voie orale selon la revendication 1, dans lequel le polymère contenant un groupe carboxyle est réticulé par le biais d'un agent de réticulation.

13. Agent à administrer par voie orale selon la revendication 12, dans lequel l'agent de réticulation est un composé métallique polyvalent.

14. Agent à administrer par voie orale selon la revendication 1, dans lequel le polymère contenant un groupe carboxyle est l'acide polyacrylique.

15. Agent à administrer par voie orale selon la revendication 6, dans lequel une teneur de la glycérine dans la couche formant un gel est désignée par A (en % en poids) et une teneur du composé à l'état solide est désignée par B (en % en poids), et une relation 0,05 ≤ B / A ≤ 1,5 est satisfaite.

16. Agent à administrer par voie orale selon la revendication 1, dans lequel le promoteur de l'absorption d'eau comprend au moins un composé parmi le polyoxyl stéarate 40 ou le polyoxyéthylène (105) polyoxypropylène (5) glycol comme glycol.
